# EUROPEAN PATENT APPLICATION

(11) **EP 1 088 534 A2**
(43) Date of publication of application: **04.04.2001**
(21) Application number: 00305533.2
(22) Date of filing: 30.06.2000
(51) Int. Cl.: A61F 5/01

(54) **Range of motion orthopaedic joint brace with a linearly actuated stop**

(30) Priority: 28.09.1999 US 156379 P
(71) Applicant: DEPUY ORTHOPAEDICS, INC., Warsaw, Indiana 46581 (US)
(72) Inventor: NISWONGER, Timothy Glenn, Monteca CA-95366 (US); ENZERINK, Robert-Jan, Davis CA-95219 (US); DUKE, Patrick James, Stockton CA-95219 (US)
(74) Representative: Belcher, Simon James

(57) **Abstract**

An orthopaedic joint brace having upper and lower struts joined by a hinge includes a linearly actuated stop mechanism operable for selectively adjustable movement thereon that acts to restrict arcuate movement of the struts about the hinge to a user selectable angular range of motion. The stop may take many forms. The stop includes an abutment that linearly translates along a strut to restrict angular motion or movement of the brace to a particular range of motion. In one form, the stop includes a stop pin that cooperates with a plurality of apertures or notches in one of the struts. The aperture or notches correspond to selected ranges of angular movement of the brace that translate to limit the range of motion thereof. In another form, the stop includes a pawl that cooperates with a plurality of notches or ratchet to set the range of angular movement.

## Description

The present invention relates to orthopaedic braces and, more particularly, to a range of motion device adaptable to orthopaedic braces used in stabilizing a joint after injury or invasive surgery.

In order to ensure the proper healing of a human joint after an injury or invasive surgery, it is often desirable to limit the pivotal motion of the human joint to a predetermined angular range between full extension and full flexion. The pivotal motion may be limited by a range of motion hinge disposed between an upper strut and a lower strut. Orthopaedic braces of this general type, including information relating to range of motion braces, and how and why such equipment is used, are disclosed in US-4655201, US-4817588, US-5292303, US-5409449, US-5658243, US-5817040, and US-5921946.

It is well known that the range of motion braces described in the aforementioned patents suffer various problems, shortcomings and disadvantages. Most notably, the range of motion hinges tend to be difficult to operate, manipulate and adjust; to be bulky; to have high profiles that tend to snag on furniture, clothes and the like; to have complicated and hard to read adjustment mechanisms; and to have loose parts.

The present invention provides a range of motion structure particularly for an orthopaedic joint brace that provides for the variable setting of the range of motion of the brace.

In one form, the present invention provides an orthopaedic joint brace that includes a first strut, a second strut, a hinge disposed between the first and second struts, and a stop carried by one of the first and second struts. The hinge allows arcuate or angular movement or motion of one of the first and/or second struts about an axis thereof corresponding to a joint axis. The stop is carried by one of the first and second struts and is movable thereon into a plurality of positions. The stop restricts the arcuate movement (i.e. determines the range of motion) of one of the first and second struts to a particular range of motion according to the position of the stop.

In another form, the present invention provides an orthopaedic joint brace that includes an upper strut, a lower strut, a hinge positioned between the upper and lower struts, a stop carried by one of the upper and lower struts, and an abutment carried by the stop. The hinge allows angular motion of one of the upper and lower struts about the hinge and an axis corresponding to a joint axis. The stop is movable on the strut toward and away from the hinge into a plurality of positions. The abutment restricts angular motion of one of the upper and lower struts to a particular range of angular motion according to a selectively adjustable position of said stop.

In another form, the present invention provides an orthopaedic joint brace including an upper strut, a lower strut, a hinge disposed between the upper strut and the lower strut, and a stop disposed on the upper strut. The hinge is adapted to allow angular motion between the upper and lower struts and provide an axis corresponding to a joint axis. The stop is linearly movable on the strut into a plurality of positions. The stop includes an abutment restricting angular motion between the upper and lower struts to a particular range of angular motion according to the linear position of the stop.

Accordingly, the present invention improves upon the prior art by providing a low-profile, range of motion hinge structure that is easily adjustable by the wearer without the use of tools. More specifically, the improved range of motion hinge structure may be adjusted without the use of tools and is lower in cost, lighter in weight, and lower in profile in comparison to the prior art. This cost-effective range of motion hinge structure allows the selective restriction of the range of motion for both extension and flexion of the joint through linearly positionable/actuated stops in an enclosed system of either locking pins and stop apertures, or by a ratchet and pawl.

The present invention will now be described by way of example with reference to the accompanying drawings, in which:
FIG. 1 is a side perspective view of an adjustable, motion-restraining knee brace containing an improved range of motion hinge assembly that embodies principles of the present invention showing the brace operatively connected to a human leg;
FIG. 2 is a side elevational view depicting the range of motion hinge of FIG. 1 showing an arbitrary degree of flexion;
FIG. 3 is a side elevational view depicting the range of motion hinge of FIG. 1 showing full degree of extension;
FIG. 4 is cross-sectional view through the range of motion hinge assembly taken along line 4-4.
FIG. 5 is a side elevational view depicting an alternative embodiment of the range of motion hinge of FIG.1 showing an arbitrary degree of flexion;
FIG. 6 is a side elevational view depicting an alternative embodiment of the range of motion hinge of FIG. 1 showing full degree of extension;
FIG. 7 is cross-sectional view through an alternative embodiment of the range of motion hinge assembly taken along line 7-7;
FIG. 8 is cross-sectional view through an alternative embodiment of the range of motion hinge assembly taken along line 8-8;
FIG. 9 is a side elevational view depicting a third embodiment of the range of motion hinge of FIG. 1 showing an arbitrary degree of flexion;
FIG. 10 is a side elevational view depicting a third embodiment of the range of motion hinge of FIG.1 showing full degree of extension.

In the drawings, corresponding reference characters indicate corresponding parts throughout the several views.

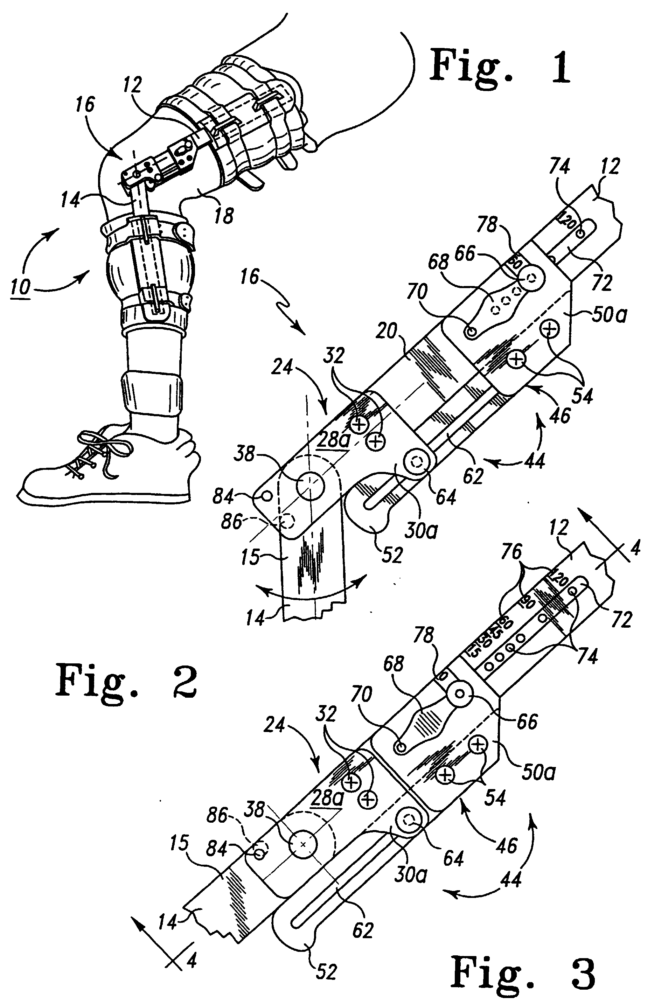

Referring to the drawings, Fig.1 shows an orthopaedic brace 10 which is adapted to fit on a human limb in order to limit the range of motion of a human joint. While it is expected that this improved orthopaedic brace is adaptable to any of the joints of the human body, such as an elbow, an ankle, or a hip, it will be described herein as used to aid the healing of the knee joint. Consequently, Fig. 1 shows the orthopaedic brace operatively connected to a human leg 18 through conventional means. Orthopaedic brace 10 includes an upper strut 12, a lower strut 14, and an improved range of motion hinge assembly 16 disposed between the upper strut 12 and the lower strut 14. While not seen in the figures, the orthopaedic brace 10 preferably includes a hinge and dual strut assembly on the opposite side of the brace and leg.

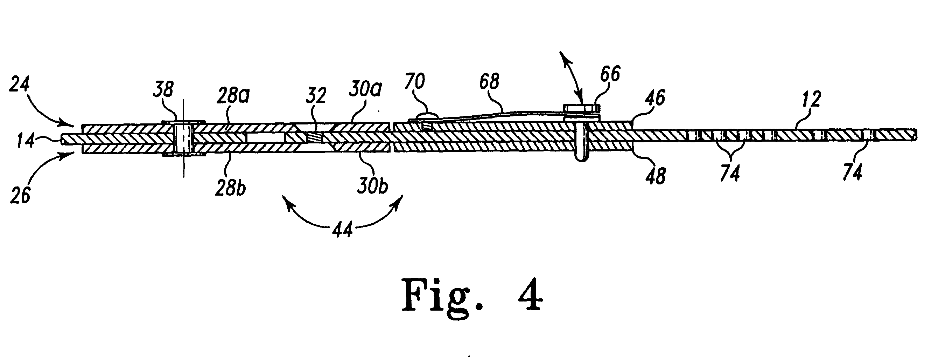

Referring to Figs. 2 and 4, the range of motion (ROM) hinge assembly or hinge assembly 16 is shown in an arbitrary degree of flexion. The hinge assembly 16 includes a pair of outer and inner opposing base plates 24 and 26, which may be identically configured. Each of the opposing base plates 24 and 26 has a generally rectangular body 28a and 28b respectively from which a respective guide-pin mounting portion 30a and 30b extends.

An end portion 20 of the upper strut 12 is sandwiched and retained between the opposing base plates 24 and 26 by means of, for example, two rivets 32, that extend into respective aligned openings at the mounting end 30 of the opposing base plates 24 and 26, and through respective aligned openings formed in the central end of the upper strut 12.

An end portion 15 of the lower strut 14 is pivotally sandwiched and retained between the opposing base plates 24 and 26 by means of a hinge pivot pin 38. The hinge pivot pin 38 extends into aligned circular openings in the centre of the body portions 28a and 28b of the respective opposing base plates 24 and 26, and an aligned circular opening formed through the end portion 15 of the lower strut 14.

The upper strut 12 is provided with a slidably attached, linearly translatable stop mechanism 44. The stop mechanism 44 comprises a second pair of opposing base plates 46 and 48, between which the upper strut 12 is disposed. The second base plates 46 and 48 are generally rectangular each with a respective connecting tab 50a and 50b appendage (of which only one connecting tab 50a is shown) that extends radially outward from the centre of the upper strut 12.

An elongated stop member 52 is sandwiched and retained between the connecting tabs 50a and 50b of each base plate 46 and 48 by means of, for example, two rivets 54, that extend down through aligned circular openings formed on the connecting tabs 50a and 50b of each respective base plate 46 and 48 and through aligned circular openings in the stop member 52. Formed longitudinally through the centre of the stop member 52 is an enclosed, elongated guide track 62 adapted to receive and retain a guide pin 64. The guide pin 64 extends down through the guide track 62 and aligned circular openings in the guide-pin mounting portions 30a and 30b of the respective base plates 24 and 26. The guide pin 64 serves to slidably attach the lower portion of the stop mechanism 44 to the upper strut 12.

Similarly, a removable stop pin 66 adjustably retains the upper portion of the stop mechanism 44 on the upper strut 12. The stop pin 66 is attached to the second outer base plate 46 by means of a flattened, elongated retaining strip 68 of flexible material, such as certain plastics or metals having these properties, which is flexibly deformable in a direction away from the face of the second outer base plate 46, but preferably not substantially deformable in the direction of elongation parallel to the face of the second outer base plate 46. As well, the elongated retaining strip 68 may be flexibly deformable in a direction toward the face of the second outer base plat 46 (e.g. a rocker switch). The end of the retaining strip 68 that is opposite the stop pin 66 is rigidly attached to the face of the second outer base plate 46 by means of, for example, a screw or rivet 70.

Referring particularly to Figs. 2 and 3, the upper strut 12 is provided with a plurality of longitudinally spaced stop apertures 74 that are inside a shallow, longitudinal track 72 formed on the face of the upper strut 12. Inscribed on the face of the upper strut 12 is a graded degree index scale 76. The depicted index scale 76 shows, as an example, an angular limit range of from 0 to 120 degrees in increments (corresponding to apertures 74) ranging from 15 to 30 degrees. The second upper base plate 46 is provided with an opening 78 adjacent to the stop pin 66 for viewing the flexion limit angle depicted on the scale 76 that corresponds to the selected stop aperture 74. It will be appreciated that the incremental angles and the maximum angles of extension and flexion may vary.

Figs. 2 to 4 illustrate the operation of the stop mechanism 44. Referring to Fig. 4, it can be seen that the stop pin 66 extends through aligned openings in the opposing base plates 46 and 48 and through one of the selectable plurality of stop apertures 74, thereby securing the upper portion of the stop mechanism 44 to the upper strut 12. By moving the stop pin 66 in a direction axially away from the upper strut 12, the stop mechanism 44 can be linearly translated up and down the longitudinal length of the upper strut 12. The amount of linear translation is defined by the length of the guide track 62, which length thereby defines the total included angle depicted on the index scale 76.

The desired limit angle is captively selected by sliding the stop mechanism 44 up or down the strut 12 to the stop aperture 74 corresponding to the angle depicted on the scale 76 and then releasing the stop pin 66 into the selected aperture 74. The retaining strip 68 will captively hold the stop pin 66 in the selected aperture 74. As the lower strut 14 is rotated toward the upper strut 12 as depicted in Fig. 2, the lower strut 14 abuts against the stop member 52, which serves to positively stop the further rotation of the lower strut 14 toward and with respect to the upper strut 12. The flexion stop angle is increased by sliding the stop mechanism 44 linearly up the upper strut 12, in a direction away from the hinge pivot pin 38, which decreases the length of the stop member 52 that the lower strut 14 may potentially abut against. Conversely, the flexion stop angle is decreased by sliding the stop mechanism 44 linearly down the upper strut 12 in a direction toward the hinge pivot pin 38, which increases the length of the stop member 52 that the lower strut 14 may potentially abut against. A 0° flexion stop angle is depicted in Fig. 3.

Extension angle is limited by the lower strut 14 abutting against an extension limit pin 84, which runs through aligned circular openings in the lower, outer corner of the body portions 28a and 28b of the respective base plates 24 and 26. The lower strut 14 may be provided at its central end with an arcuate-shaped notch 86 adapted to mate with the extension limit pin at full extension.

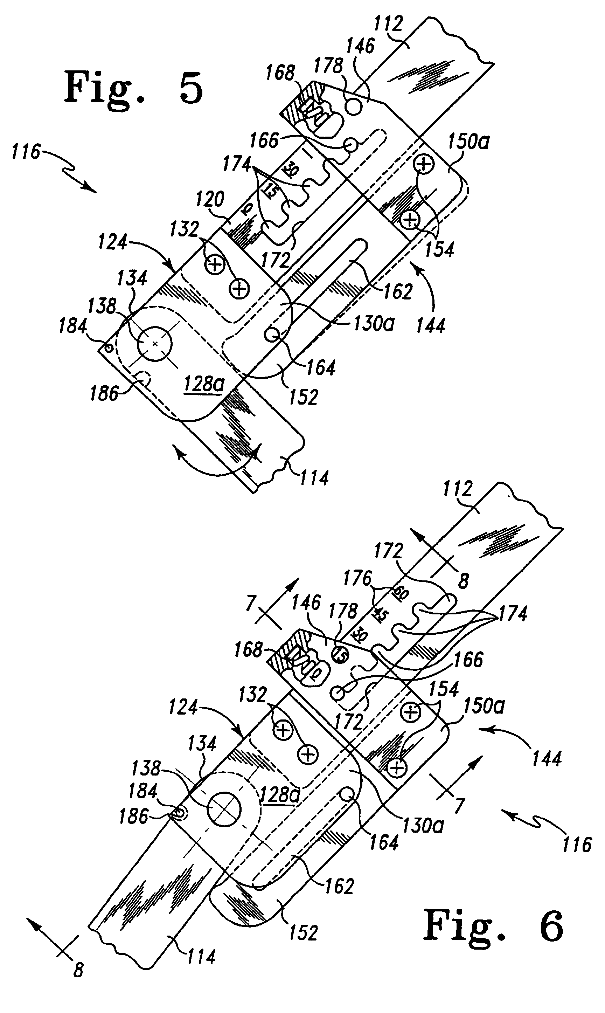

Referring to Fig. 5, an alternative embodiment is shown in an arbitrary degree of flexion. The alternative hinge assembly 116 includes a pair of outer and inner opposing base plates 124 and 126, which may be identically configured. Each of the opposing base plates has a generally rectangular body 128a and 128b from which a respective guide-pin mounting portion 130a and 130b extends (of which only guide pin mounting portion 130a may be seen).

An end portion 120 of the upper strut 112 is sandwiched and retained between the opposing base plates 124 and 126 by means of, for example, two rivets 132, which are extended down into aligned openings at the mounting ends 130a and 130b of the respective opposing base plates 124 and 126, and through aligned openings formed in the central end of the upper strut 112.

An end portion 134 of the lower strut 114 is pivotally sandwiched between the opposing base plates 124 and 126, by means of a hinge pivot pin 138 that extends into aligned circular openings in the centre of the body portions 128a and 128b of the opposing base plates 124 and 126, and an aligned circular opening formed through the end portion 134 of the lower strut 114.

The upper strut 112 has formed through its centre a longitudinal stop channel 172. The upper strut 112 is provided with a slidably attached, linearly translatable stop mechanism 144. The stop mechanism 144 comprises a second pair of opposing base plates 146 and 148, between which the upper strut 112 is disposed. The second base plates 146 and 148 are generally rectangular each with a connecting tab 150a and 150b appendage that extends radially outward from the centre of the upper strut 112.

An elongated stop member 152 is sandwiched and retained between the connecting tabs 150a and 150b of the respective base plates 146 and 148 by means of, for example, two rivets 154, that extend down through aligned circular openings formed on the connecting tabs 150a and 150b of the respective base plates 146 and 148 and through aligned circular openings on the stop member 152, and two rivets (not shown) 194, which extend down through aligned circular openings 196 (not shown) in base plates 146 and 148.

Formed longitudinally through the centre of the stop member 152 is an enclosed, elongated guide track 162 adapted to receive and retain a guide pin 164, that extends down through the guide track 162 and aligned circular openings in the guide-pin mounting portions 130a and 130b of the respective base plates 124 and 126. The guide pin 164 serves to slidably attach the lower portion of the stop mechanism 144 to the upper strut 112.

A stop pin 166, which extends down through aligned openings in the base plates 146 and 148, is disposed within the stop channel 172, and is adapted to engage a plurality of stop notches 174 formed along the edge of the stop channel 172 furthest away from the stop member 152.

Inscribed on the face of the upper strut 112 is a graded degree index scale 176. The depicted index scale 176 shows, as an example, an angular limit range of from 0 to 120 degrees in increments ranging from 15 to 30 degrees. The second upper base plate 146 is provided with an opening 178 adjacent to the stop pin 166 for viewing the flexion limit angle depicted on the scale 176 that corresponds to the selected stop notch 174. It will be appreciated that the incremental angles and the maximum angles of extension and flexion may vary.

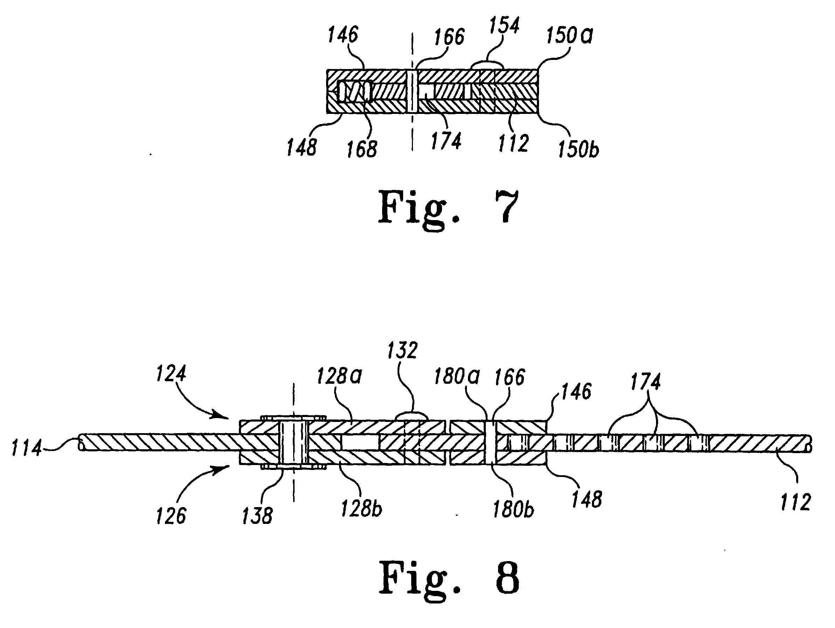

Figs. 5 to 8 illustrate the operation of the stop mechanism 144. Referring to Figs. 7 and 8, it can be seen that the stop pin 166 extends through aligned openings 180a and 180b in the opposing base plates 146 and 148 respectively and through the stop channel 172. Disposed between the base plates 146 and 148 is a spring means 168 that is perpendicularly juxtaposed between the upper strut 112 and the base plates 146 and 148 in the transverse plane. The spring means 168 acts against the edge of upper strut 112 to urge the stop pin 166 to captively engage one of the selected plurality of stop notches 174. Compressing the spring 168 disengages the stop pin 166 to allow linear translation of the stop mechanism 144 up and down the upper strut 112 within the complementary limits of the longitudinal stop channel 172 and the guide track 162.

The lengths of the stop channel 172 and the guide track 162 translate into the total included angle depicted on the index scale 176. The desired limit angle is captively selected by sliding the stop mechanism 144 up or down the upper strut 112 to the stop aperture 174 corresponding to the angle depicted on the scale 176. As the lower strut 114 is rotated toward the upper strut 112 as depicted in Fig. 5, the lower strut 114 abuts against the stop member 152, which serves to positively stop the further rotation of the lower strut 114 toward and with respect to the upper strut 112.

The flexion stop angle is increased by sliding the stop mechanism 144 linearly up the upper strut 112, in a direction away from the hinge pivot pin 138, which decreases the length of the stop member 152 that the lower strut 114 may potentially abut against. Conversely, the flexion stop angle is decreased by sliding the stop mechanism 144 linearly down the upper strut 112 in a direction toward the hinge pivot pin 138, which increases the length of the stop member 152 that the lower strut 114 may potentially abut against.

Extension angle is limited by the lower strut 114 abutting against an extension limit pin 184, which runs through aligned circular openings in the lower, outer corner of the body portions 128a and 128b of the base plates 124 and 126 respectively. The lower strut 114 may be provided at its central end with an arcuate-shaped notch 186 adapted to mate with the extension limit pin 184 at full extension.

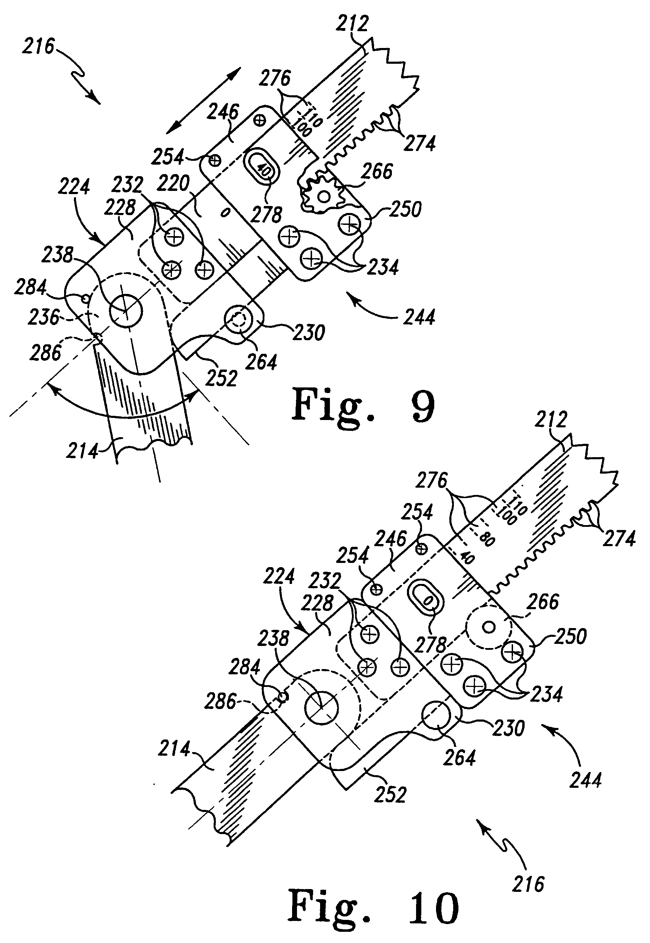

Referring to Fig. 9, another alternative embodiment is shown in an arbitrary degree of flexion. The alternative hinge assembly 216 includes a pair of outer and inner opposing base plates in similar manner to the previous embodiments, of which only one base plate 224 can be seen in the figures, which may be identically configured. Each of the opposing base plates has a generally rectangular body 228 from which a guide-pin mounting portion 230 extends. An end portion 220 of the upper strut 212 is sandwiched and retained between the opposing base plates by means of, for example, a plurality rivets 232, which are extended down into aligned openings of the opposing base plates, and through aligned openings formed in the end of the upper strut 212. An end portion 236 of the lower strut 214 is pivotally sandwiched between the opposing base plates, by means of a hinge pivot pin 238 that extends into aligned circular openings in the centre of the body portions 228 of opposing base plates, and an aligned circular opening formed through the end portion 236 of the lower strut 214.

The upper strut 212 is provided with a slidably attached, linearly translatable stop mechanism 244. The stop mechanism 244 comprises a second pair of opposing base plates in similar manner to the previous embodiments, of which only one base plate 246 can be seen in the figures, between which the upper strut 212 is disposed. The second opposing base plates are generally rectangular each with a connecting tab 250 appendage that extends radially outward from the centre of upper strut 212. Between the connecting tabs 250 of each opposing base plate is sandwiched an elongated stop member 252 by means of, for example, a plurality of rivets 234, 254, which extend down through aligned circular openings formed on the connecting tabs 250 of each base plate and through aligned circular openings on the stop member 252, and two rivets (not shown) 294, which extend down through aligned circular openings 296 (not shown) in the base plates (246). A guide pin 264 extends down through circular openings in the guide-pin mounting portion 230 of each base plate, and serves to prevent the stop member 252 from rotating out and away from the struts 212 and 214.

The upper strut 212 has formed along its side edge that is proximate to the stop mechanism 244 a longitudinally disposed gear rack 274 adapted to function with a gear 266 that is sandwiched between the base plates by means of, for example, a rivet that extends down through circular openings in each base plate and through a circular opening through the centre of the gear 266. The stop assembly 244 is linearly translated up and down the longitudinal length of the upper strut 212 through the cooperative interaction of the teeth of the gear 266 and the gear rack 274.

The length of the gear rack 274 defines the limits of movement of the stop assembly 244, and, thereby, the flexion limit angle. Inscribed on the face of the upper strut 212 is a graded degree index scale 276 that reflects the total included flexion limit angle defined by the length of the gear rack 274. The depicted index scale 276 shows, as an example, an angular limit range of from 0 to 110 degrees in increments ranging from 10 to 30 degrees. The second upper base plate 246 is provided with an opening 278 for viewing the flexion limit angle depicted on the scale 276. It will be appreciated that the incremental angles and the maximum angles of extension and flexion may vary.

The desired limit angle is selected by sliding the stop mechanism 244 up or down the upper strut 212. As the lower strut 214 is rotated toward the upper strut 212 as depicted in Fig. 9, the lower strut 214 abuts against the stop member 252, which serves to positively stop the further rotation of the lower strut 214 toward and with respect to the upper strut 212. The flexion stop angle is increased by sliding the stop mechanism 244 linearly up the upper strut 212, in a direction away from the hinge pivot pin 238, which decreases the length of the stop member 252 that the lower strut 214 may potentially abut against. Conversely, the flexion stop angle is decreased by sliding the stop mechanism 244 linearly down the upper strut 212 in a direction toward the hinge pivot pin 238, which increases the length of the stop member 252 that the lower strut 214 may potentially abut against.

Extension angle is limited by the lower strut 214 abutting against an extension limit pin 284, which runs through aligned circular openings in the lower, outer corner of the body portions 228 of the base plates. The lower strut 214 may be provided at its central end with an arcuate-shaped notch 286 adapted to mate with the extension limit pin at full extension.

While not depicted, each of the three described embodiments could include an additional linear stop mechanism on the opposite side of the hinge assembly adapted to selectively limit the angle of extension in a manner approximating the described manner in which the angle of flexion is limited. As well, it should be appreciated that functionality and operability of the upper and lower (or first and second) struts and their features are interchangeable as well as the stop mechanism.

Although the invention has been described in detail with reference to certain preferred embodiments, variations and modifications exist within the scope and spirit of the invention. Additional features of the invention will become apparent to those skilled in the art upon consideration of the detailed description of preferred embodiments exemplifying the best mode of carrying out the invention as presently perceived.

## Claims

1. An orthopaedic joint brace comprising:
a first strut;
a second strut;
a hinge disposed between the first and second struts and configured to allow arcuate movement of one of the first and second struts about an axis corresponding to a joint axis; and
a stop carried by one of the first and second struts and positionable at any one of a plurality of positions, the stop being configured to restrict arcuate movement of one of the first and second struts to a particular range of motion according to the position of the stop.

2. A brace as claimed in claim 1, in which the stop includes a stop pin for selectively positioning the stop into the one of the plurality of positions.

3. A brace as claimed in claim 2, in which the one of the first and second struts carrying the stop includes a plurality of apertures corresponding to the plurality of positions for receiving the stop pin.

4. A brace as claimed in claim 3, in which the stop pin is removably biassed into a selective one of the plurality of apertures to selectively position the stop.

5. A brace as claimed in claim 4, in which the stop pin is removably biassed by a leaf spring, the one of the first and second struts carrying the stop includes a scale indicating a total range of arcuate motion of the one of the first and second struts, and the stop includes a viewer indicating the particular range of arcuate motion of the first and second struts on the scale according to the position of the stop.

6. A brace as claimed in claim 2, in which the one of the first and second struts carrying the stop includes a plurality of notches corresponding to the plurality of positions for engagement by the stop pin.

7. A brace as claimed in claim 6, in which the stop pin is removably biassed into engagement with a selective one of the plurality of notches to selectively position the stop.

8. A brace as claimed in claim 7, in which the stop pin is fixed to the stop and the stop is biassed by a spring operable between the stop and the one of the first and second struts carrying the stop for removably positioning the stop pin in a selective one of the plurality of notches.

9. A brace as claimed in claim 1, in which the one of the first and second struts carrying the stop includes a plurality of notches corresponding to the plurality of positions and the stop includes a pawl operable to selectively engage some of the plurality of notches.

10. An orthopaedic joint brace comprising:
an upper strut;
a lower strut;
a hinge positioned between the upper and lower struts and configured to allow angular motion of one of the upper and lower struts about an axis corresponding to a joint axis;
a stop carried by one of the upper and lower struts and movable thereon toward and away from the hinge into one of a plurality of positions; and
an abutment carried by the stop and configured to restrict angular motion of one of the upper and lower struts to a particular range of angular motion according to a selectively adjustable position of the stop.

11. A brace as claimed in claim 10, in which the stop includes a stop pin for selectively positioning the stop into the one of the plurality of positions, and the one of the upper and lower struts carrying the stop includes a plurality of apertures corresponding to the plurality of positions for receiving the stop pin.

12. A brace as claimed in claim 11, in which the stop pin is removably biassed into a selective one of the plurality of apertures to selectively position the stop by a leaf spring.

13. A brace as claimed in claim 11, in which the one of the upper and lower struts carrying the stop includes a plurality of notches corresponding to the plurality of positions for engagement by the stop pin, and the stop pin is removably biassed into engagement with a selective one of the plurality of notches to selectively position the stop.

14. A brace as claimed in claim 13, in which the stop pin is fixed relative to the stop and the stop is biassed by a spring operable between the stop and the one of the upper and lower struts carrying the stop for removably positioning the stop pin in a selective one of the plurality of notches.

15. A brace as claimed in claim 10, in which the one of the upper and lower struts carrying the stop includes a plurality of notches corresponding to the plurality of positions and the stop includes a pawl operable to selectively engage some of the plurality of notches.

16. An orthopaedic joint brace comprising:
an upper strut;
a lower strut;
a hinge disposed between the upper strut and the lower strut and configured to allow angular motion between the upper and lower struts about an axis corresponding to a joint axis; and
a stop disposed on the upper strut and linearly movable thereon into one of a plurality of positions, the stop including an abutment configured to restrict angular motion between the upper and lower struts to a particular range of angular motion corresponding to the linear position of the stop.

17. A brace as claimed in claim 16, in which the stop includes a stop pin for selectively positioning the stop into the one of the plurality of positions, and the upper strut includes a plurality of apertures corresponding to the plurality of positions for receiving the stop pin.

18. A brace as claimed in claim 17, in which the upper strut includes a plurality of notches corresponding to the plurality of positions for engagement by the stop pin, and the stop pin is removably biassed into engagement with a selective one of the plurality of notches to selectively position the stop.

19. A brace as claimed in claim 18, in which the stop pin is fixed relative to the stop and the stop is biassed by a spring operable between the stoop and the upper strut for removably positioning the stop pin in a selective one of the plurality of notches.

20. A brace as claimed in claim 16, in which the upper strut includes a plurality of notches corresponding to the plurality of positions and the stop includes a pawl operable to selectively engage some of the plurality of notches.
